# EUROPEAN PATENT APPLICATION

(11) **EP 1 847 604 A1**
(43) Date of publication of application: **24.10.2007**
(21) Application number: 06290641.7
(22) Date of filing: 19.04.2006
(51) Int. Cl.: C12N 15/10, C12Q 1/68, G01N 33/542

(54) **Intracellular method for selecting a peptide or polypeptide which binds to a bait peptide or polypeptide**

(71) Applicant: Millegen, 31670 Labege (FR)
(72) Inventor: Renaut, Laurence, 31290 Villefranche de Lauragais (FR); Bouayadi, Khalil, 31520 Ramonville Saint-Agne (FR)
(74) Representative: Nargolwalla, Cyra

(57) **Abstract**

The present invention provides a versatile and sensitive method for screening/studying interaction of two peptides or polypeptides A and B within the cytoplasm of a host cell. The method is based on the use of two distinct chimeric polypeptides. The first chimeric polypeptide containing an aggregation domain fused to a polypeptide A and the second one containing a phenotype-associated functional domain fused to a second polypeptide B. When these chimeric polypeptides are co-expressed within a host cell allowing aggregation of the aggregation domain, the phenotype of said host cell depends on the entrapment of the phenotype-associated functional domain which occurs when A and B interact with each other.

## Description

### FIELD OF THE INVENTION

This invention relates to kits and methods for selecting a peptide or polypeptide which binds to a bait peptide or polypeptide.

### BACKGROUND OF THE INVENTION

By the end of the human genome sequencing project, several open reading frames coding for new proteins were identified. This finding allowed biological research to focus on the characterisation of these new proteins, involving investigation or identification of interacting protein partners, chemical compounds which disturb protein interaction and specific antibodies for targeting protein.

For this purpose, methods allowing studies of protein/protein interaction have been developed:
Different in vitro methods for protein/protein interaction studies such as immunoprecipitation, pull down assays, TAP (Tandem Affinity Purification) and protein array are based on the isolation of interacting proteins. In these methods, proteins of interest, tagged or not, are directly or indirectly immobilised and proteins having interaction properties are retained on their targets.
The major drawback of these methods is that after its isolation, the candidate protein for interaction with the protein of interest must be characterised. For this purpose, long and cost effective methods like 2D SDS-page and mass spectroscopy characterisation have to be used.
In order to facilitate the characterisation of the putative interacting partner, methods allowing the association of candidate protein with their own nucleotide counterparts have been developed.
During the last twenty years, many display methods have been developed and among them, phage display, yeast display, and bacterial display are widely used for screening studies. In these methods, a peptide or a protein is expressed at the surface of the cell or of the virus containing the counterpart genetic information. When interaction occurs, the structure containing the counterpart nucleotide sequence is retained with the target protein and after several cycles of binding, the polypeptide of interest linked with its counterpart nucleotide sequence is enriched and can be characterised. These techniques allow large protein libraries to be screened against a bait protein. However, the bait protein has to be purified. This is a major drawback because purification of proteins can often be very problematic.

Methods allowing the detection of intracellular interaction have been developed. Within these methods, the bait protein is expressed in the cell and purification steps are not required.
The yeast two hybrid method (cf. Fields and Song, 1989 and WO0200729) is based on the transcription of a reporter gene following a protein/protein interaction between two hybrid-proteins. Thus, bait and putative interacting protein partners are fused respectively with the DNA binding domain and the transcription activation domain of a transcription factor. When the interaction occurs, the physical joining of these two domains allows the functional restoration of the transcription factor and the reporter gene expression. However large numbers of controls are required in order to reduce the number of false positive results. Furthermore interactions occur in the nucleus and not all proteins can be studied with this system.
To overcome this drawback, several techniques have been developed in order to highlight protein/protein interaction occurring in the cytoplasm of cells. Among them, the cyto Trap method (cf. Broder et al, 1998 and US5776689) and the protein fragment complementation (PCA) method (cf. Johnsson and Varshavsky, 1994 and WO9529195) In the Cyto Trap method, cytoplasmic protein/protein interaction induces oncogene RAS activation and growth of a temperature-sensitive cdc25 yeast mutant. Briefly, a bait protein is located in the membrane and proteins (library) are fused to the human SOS protein which is able to activate RAS. Interaction with the bait protein activates the RAS pathway and thereby confers on the yeast the ability of growing at 37°C. A major drawback of this strategy is that the screening is based on a single mutation in the cdc25 gene, and reversion of the genotype can give false positives.
Protein fragment complementation assays (PCAs) were first implemented in the Split-ubiquitin system. This method is based on the re-assembly of N-and C-terminal halves of ubiquitin (Nub and Cub) fused to interacting proteins. The reassembled "native" ubiquitin is recognised and cleaved by ubiquitin specific protease. A reporter protein is released when interaction occurs. Derivative methods have also been described. The common principle of these techniques is that the reporter protein itself is split into two fragments. They are fused to the potential interaction partners and the interaction of the proteins restores the function of the cleaved reporter. Reporter proteins used for this purpose are enzymes such as beta-galactosidase (Rossi et al, 1997 and WO9844350), beta-lactamase (Wehrman et al, 2002 and WO03058197), or reporter fluorescent proteins such as GFP or YFP (Remy, 2004 and WO2004092336). In these methods, the bait protein is genetically fused to another protein.
A major drawback of this system is that controls cannot be performed in order to ensure the functional integrity of this protein when fused to the bait. For example, in the Yeast two hybrid method, when the bait is fused to a DNA binding domain or a transcription activation domain, it can disable the function of the DNA binding domain or of the transcription activation domain. In this case, even if interaction occurs, the reporter gene is not transcribed.

### SUMMARY OF THE INVENTION

The present invention provides a versatile and sensitive method for selecting a peptide or polypeptide which binds to a bait peptide or polypeptide within the cytoplasm of a host cell.

The invention is based on the aggregating property of certain protein domains called aggregation domains. When a peptide or a polypeptide is fused to such an aggregation domain, the chimeric polypeptide obtained when expressed in a suitable host cell where the aggregation domain can form aggregates will form part of such an aggregate. A polypeptide interacting with such a chimeric polypeptide which forms part of a protein aggregate will be entrapped within said aggregate.
The present invention is based on the use of two distinct chimeric polypeptides. The first chimeric polypeptide contains an aggregation domain fused to a polypeptide A and the second contains a phenotype-associated functional domain fused to a second polypeptide B. When these chimeric polypeptides are co-expressed within a host cell allowing aggregation of the aggregation domain, the phenotype of said host cell depends on the entrapment of the phenotype-associated functional domain, which occurs when A and B interact with each other.

### DETAILED DESCRIPTION OF THE INVENTION

The present invention provides a kit for selecting a peptide or polypeptide A which binds to a peptide or polypeptide B comprising:
- a first expression vector comprising a nucleotide sequence encoding a first chimeric polypeptide of formula X-A comprising an aggregation domain, which enables the aggregation in a host cell of said chimeric polypeptide
   wherein X is a polypeptide or a peptide and A is a polypeptide or peptide; and
- a second expression vector comprising a nucleotide sequence encoding a second chimeric polypeptide of formula B-Y, wherein B is a polypeptide or peptide and Y is a polypeptide or a peptide, comprising a phenotype-associated functional domain,
   wherein when B-Y and X-A are expressed in a host cell the phenotype of said host cell depends on whether B-Y is entrapped or not in an aggregate, B-Y being entrapped if A and B interact with each other.

The two different components of the chimeric polypeptide of formula X-A or B-Y can be directly linked or linked via a spacer comprised of a peptide of 1 to 20 amino acids. X or B may be linked to the N-terminal or to the C-terminal amino-acid of A and Y respectively.

In a specific embodiment of the present invention, if the polypeptide A already comprises an aggregation domain, which enables aggregation in a host cell of said polypeptide A, the presence of a further aggregation domain may not be needed. In this specific case, a suitable first expression vector may be an expression vector comprising a nucleotide sequence encoding a polypeptide A comprising an aggregation domain, which enables the aggregation in a host cell.

Typically A or B may be expression products issued from a cDNA library or from a library resulting from the construction of nucleotide sequences repertories, said nucleotide sequences being characterised in that said nucleotides differ by at least one base. The generation of the variant nucleotide sequences encoding A or B may be performed by site-directed mutagenesis, preferentially by random mutagenesis.

In a preferred embodiment, A or B is an immunoglobulin, or a member of the immunoglobulin super-family, or any fragment thereof. In this context, the term immunoglobulin includes members of the classes IgA, IgD, IgE, IgG, and IgM. The term immunoglobulin super-family refers to all proteins which share structural characteristics with the immunoglobulins, including, for example, the T-cell receptor, or any of the molecules CD2, CD4, CD8 etc. Also included are fragments which can be generated from these molecules, such as Fv (the two variable regions), single chain Fv (an Fv complex in which the component chains are joined by a linker molecule), Fab, F(ab')₂ or an immunoglobulin domain, such as the constant fragment (Fc), the variable heavy chain domain (VH) or the variable light chain domain VL.

Typically a kit according to an embodiment of the present invention further comprises a host cell wherein when said first and second expression vectors are introduced into said host cell, said host cell expresses chimeric polypeptides X-A and B-Y and X-A forms part of a protein aggregate which is present in said host cell and the phenotype of said host cell depends if B-Y is entrapped or not in said aggregate.
Typically said host cell is a prokaryotic or an eukaryotic cell, preferably a yeast cell or a mammalian cell.
Typically the aggregation domain is naturally able to form aggregate within said host cell. Alternatively the skilled person may use existing protocols such as the ones developed for studying prions to transform a host cell in such a way that the host cell expresses polypeptides which promotes the formation of protein aggregates within the host cell.
It falls within the ability of the skilled person to select or engineer a polypeptide or a peptide X, comprising an aggregation domain, which enables the aggregation in a host cell of said chimeric polypeptide. The skilled person may for example select a polypeptide or a peptide X comprising an aggregation domain which is already present in said host cell. Aggregation domains within prion proteins are well known (see for review Chernoff, 2004). Furthermore it has been demonstrated that the ability of forming protein aggregate can be conferred to a protein by adding a very short amino acid stretch (see for example Ventura et al., 2004).
Typically X comprises an aggregation domain selected from the group consisting of the Sup35 amino-terminal domain, a domain which is rich in glutamine and asparagines, a domain consisting of 50 to 400 glutamine residues and an intracellular insoluble domain.
Intracellular insoluble domains form aggregate due to their insolubility.
Typically X is an amyloidogenic polypeptide. Examples of amyloidogenic polypeptides are prion proteins (e.g. Sup35, New1 Ure2, PrP), huntingtin, alpha-syneclein, beta-amyloid peptide or fragments and derivatives thereof having aggregating properties.
By fragment and derivative thereof it is meant a polypeptide, which is obtainable by substitution, deletion and/or addition of one or several amino acids in the original amino acid sequence.
It falls within the ability of the skilled person to select a polypeptide or a peptide Y comprising a phenotype-associated functional domain, wherein when B-Y is expressed in a host cell the phenotype of said host cell depends if B-Y is entrapped or not in a protein aggregate, B-Y being entrapped if A and B interact with each other.
Typically Y is a polypeptide working with poorly diffusible or specifically located substrates (e.g. polyosomes in the case of Sup35). The sequestration within a protein aggregate of such a polypeptide results in decreased function due to decreased access to a substrate. It falls within the ability of the skilled person to select or engineer a host cell in which the decreased function is associated with a modification of the phenotype of said host cell. For this purpose the skilled person may use one or more reporter proteins.
Typically Y may be selected from the group consisting of a translation termination factor, a transcription factor and an enzyme.

In one embodiment of the present invention, a kit is provided, which comprises:
a first expression vector comprising a nucleotide sequence encoding a first chimeric polypeptide of formula X-A as described previously; and
a second expression vector comprising a cloning site which enables the introduction of a nucleotide sequence encoding a peptide or polypeptide B in such a way that the chimeric polypeptide of formula B-Y as described previously is expressed in a host cell when said expression vector is introduced into said host cell.

In one embodiment of the present invention, a kit is provided, which comprises:
a first expression vector comprising a cloning site which enables the introduction of a nucleotide sequence encoding a peptide or polypeptide A in such a way that the chimeric polypeptide of formula X-A as described previously is expressed in a host cell when said expression vector is introduced into said host cell; and
a second expression vector comprising a cloning site which enables the introduction of a nucleotide sequence encoding a peptide or polypeptide B in such a way that the chimeric polypeptide of formula B-Y as described previously is expressed in a host cell when said expression vector is introduced into said host cell.

In one embodiment of the present invention, a kit is provided, which comprises:
a first expression vector comprising a cloning site which enables the introduction of a nucleotide sequence encoding a peptide or polypeptide A in such a way that the chimeric polypeptide of formula X-A as described previously is expressed in a host cell when said expression vector is introduced into said host cell; and
a second expression vector comprising a nucleotide sequence encoding a second chimeric polypeptide of formula B-Y as described previously.

In another embodiment of the present invention a kit is provided, which comprises:
a first expression vector comprising a nucleotide sequence encoding a first chimeric polypeptide of formula X-A as described previously or a first expression vector comprising a cloning site which enables the introduction of a nucleotide sequence encoding a peptide or polypeptide A in such a way that the chimeric polypeptide of formula X-A is expressed in a host cell when said expression vector is introduced into said host cell; and
a library of second expression vectors each comprising a nucleotide sequence encoding a chimeric polypeptide of formula B-Y as described previously, wherein B is a polypeptide or peptide which varies within the library.

In another embodiment of the present invention a kit is provided, which comprises:
a library of first expression vectors, each comprising a nucleotide sequence encoding a first chimeric polypeptide of formula X-A as described previously, wherein A is a polypeptide or peptide which varies within the library; and
a second expression vector comprising a nucleotide sequence encoding a second chimeric polypeptide of formula B-Y as described previously or a second expression vector comprising a cloning site which enables the introduction of a nucleotide sequence encoding a peptide or polypeptide B in such a way that the chimeric polypeptide of formula B-Y is expressed in a host cell when said expression vector is introduced into said host cell.

Typically the libraries result from the construction of nucleotide sequences repertories, nucleotide sequences characterised in that they are different by at least one change. The generation of the variant nucleotide sequences encoding A or B may be performed by site-directed mutagenesis, preferentially by random mutagenesis. Random mutagenesis can be performed by using a mutase, Pol beta for example (see WO0238756).

In another embodiment of the present invention a host cell is provided, which enables the expression of the first and the second expression vector as described previously. Typically said host cell is a prokaryotic or an eukaryotic cell, preferably a yeast cell or a mammalian cell.
Typically the expression vectors are introduced into the host cell by standard co- or sequential transformation methods.
Alternatively, if the host cell is a yeast cell, mating may be used in order to improve the cotransformation efficiency.
In a further embodiment of the present invention, a kit is provided, which comprises:
a first haploid host yeast cell comprising the first expression vector as described previously; and
a second haploid host yeast cell comprising the second expression vector as described previously,
wherein said first and said second haploid yeast host cells are of opposite mating type.

In another embodiment of the present invention a kit is provided, which comprises:
a first haploid host yeast cell comprising the first expression vector as described previously; and
a library of second haploid host yeast cells, each comprising the second expression vector encoding a chimeric polypeptide of formula B-Y as described previously, wherein B is a polypeptide or peptide which varies within the library,
wherein said first and said second haploid host cells are of opposite mating type.

In another embodiment of the present invention a kit is provided, which comprises:
a library first haploid host yeast cell, each comprising the first expression vector as described previously, wherein A is a polypeptide or peptide which varies within the library; and
a second haploid yeast host cells comprising the second expression vector encoding a chimeric polypeptide of formula B-Y as described previously; wherein said first and said second haploid host yeast cells are of opposite mating type.

An example of suitable host yeast cell for carrying out the present invention when Y is a translational termination factor, is a host yeast cell containing a nonsense mutation in the *ade1* gene or in another gene in which the nonsense mutation induces a phenotype switch.

In another embodiment of the present invention, a method for selecting a peptide or polypeptide B which binds to a peptide or polypeptide A is provided. A or B may be used as bait. Typically for performing this method the skilled person may use the kits and host cells described previously.

In one embodiment of the present invention, a method for selecting a peptide or polypeptide B which binds to a bait peptide or polypeptide A is provided, which comprises the steps of:
a) generating a library of host cells, each expressing the two chimeric polypeptides X-A and B-Y as described previously and wherein B is a polypeptide or peptide which varies within the library;
b) detecting a change of phenotype within the host cells of the library;
c) isolating from the host cells with a modified phenotype the expression vector encoding B-X;
d) determining the nucleotide sequence encoding B from the expression vector isolated in step c).

In an alternative embodiment of the present invention, a method for selecting a peptide or polypeptide A which binds to a bait peptide or polypeptide B is provided, which comprises the steps of:
a) generating a library of host cells, each expressing the two chimeric polypeptides X-A and B-Y as described previously and wherein A is a polypeptide or peptide which varies within the library;
b) detecting a change of phenotype within the host cells of the library;
c) isolating from the host cells with a modified phenotype the expression vector encoding X-A;
d) determining the nucleotide sequence encoding A from the expression vector isolated in step c).

Once the nucleotide sequence of B or A is determined, B or A can be easily produced.

One of the advantages of these methods is the reduced number of false positive results. Easy control can be performed. The presence of X-A in an aggregate can easily be assessed and the effect of the presence of B on the phenotype-associated functional domain also.
Typically in the method for selecting a peptide or polypeptide B which binds to a bait peptide or polypeptide A, in order to generate a library of host cells, each expressing the two chimeric polypeptides X-A and B-Y, the following steps may be followed:
i) generating a library of host cells, expressing the chimeric polypeptides B-Y, wherein B is a polypeptide or peptide which varies within the library;
ii) selecting the host cells having the phenotype-associated with Y;
iii) transforming the host cells selected in such a way that they express the chimeric polypeptides X-A as described previously.

Typically in the method for selecting a peptide or polypeptide A which binds to a bait peptide or polypeptide B, the following control may be performed:
B-Y is expressed in a host cell in order to check if when Y fused to B, the phenotype-associated functional domain of Y is still active. Typically the method comprises the following steps:
   i) generating host cell, expressing the chimeric polypeptide B-Y, wherein B is a bait peptide.
   ii) Selecting host cells having the phenotype associated with Y.
   iii) Transforming the selected host cells in such a way that a library of host cells is generated, each expressing the two chimeric polypeptides X-A and B-Y wherein A is a polypeptide or peptide which varies within the library.

In contrast with Yeast two hybrid method, the control performed in step ii), enables the confirmation before screening that the functional domain Y is able to ensure its function in a fusion context.

In another embodiment of the present invention a method for screening compounds interfering with the interaction of two polypeptides A and B is provided, which comprises the steps of:
a) adding the compound to be screened to a host cell expressing the two chimeric polypeptides X-A and B-Y as described previously;
b) detecting a change of phenotype of said host cell.

Typically if the compounds to be screened do not easily cross the cytoplasmic membrane of the host cell, the skilled person may use existing protocols such as cell permeabilization techniques in order to facilitate the entry into the cell of the compounds.

In the following, the invention will be illustrated by means of the following examples as well as the figures.

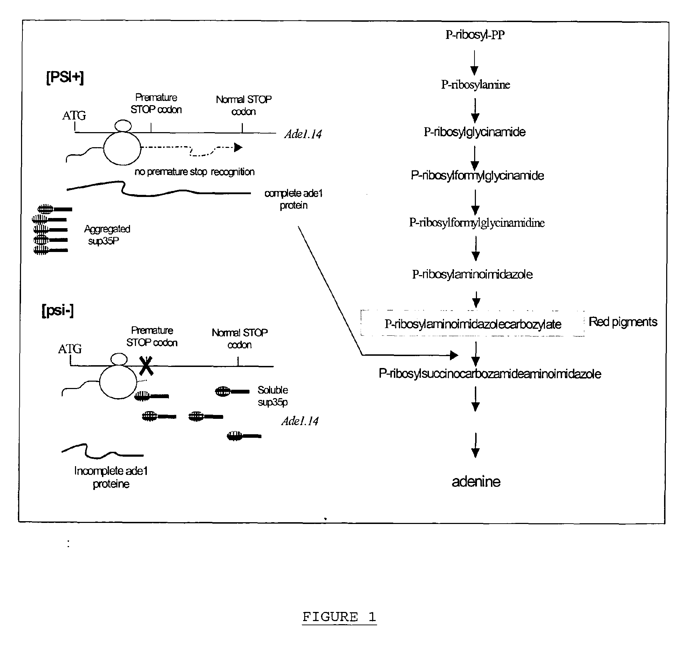

Figure 1 shows the general mechanism which occurs in yeast 74D694 strain explaining the [PSI+] and [psi-] phenotypes.

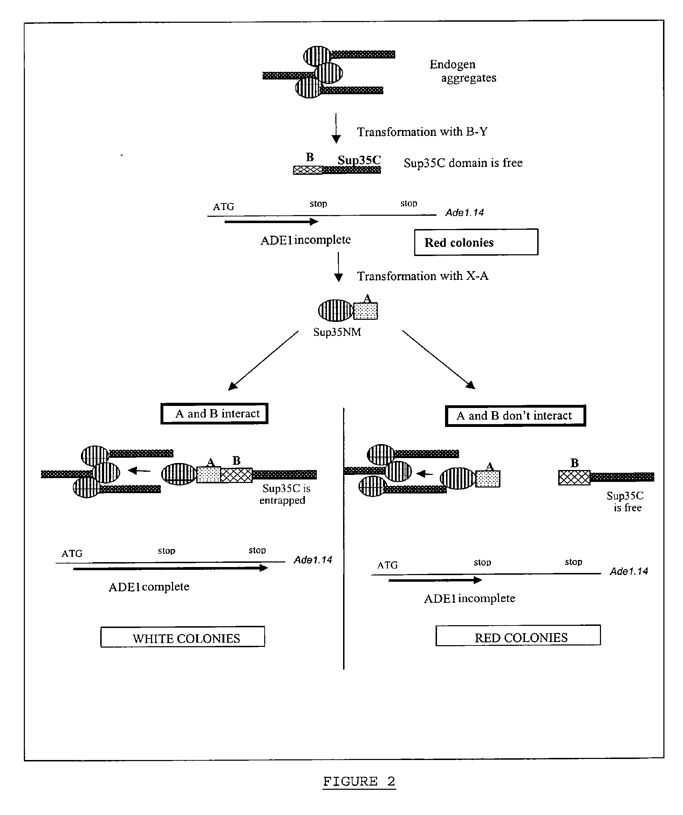

Figure 2 is a schematic representation of the molecular events occurring in a yeast cell where endogen sup35 aggregates are present, leading to a change of phenotype. The method is exemplified with the protein sup35 (abbreviated as sup35 or sup35p). In the present case X is sup35NM and Y is sup35C.
Figure 3 illustrate different constructs that allow expression of different fusion proteins used in the examples.
Figure 4 is a schematic representation of the mutated lacZ gene. The mutation is represented (circle) and the SpeI and EcoRV restriction sites used to construct the yeast expression plasmid pTEF-lacZ-mut are also represented.
Figure 5 are pictures of petri dishes with colonies transformed with lacZ or lacZmut gene platted on X-gal containing medium (A) or X-gal + GuHCl containing medium (B).
Figures 6 and 7 are pictures of colonies transformed with different constructs.

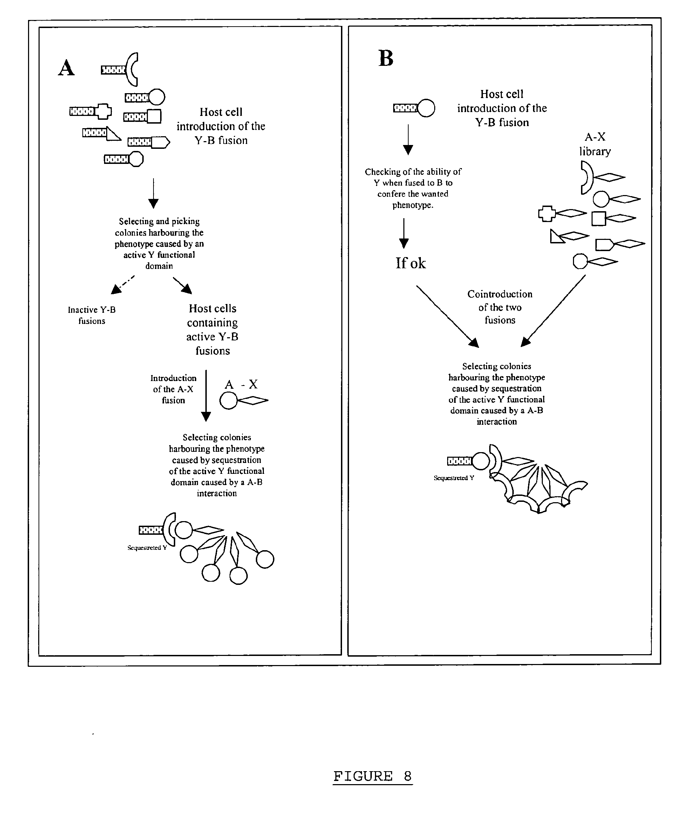

Figures 8A and 8B are schematic representations of examples of suitable strategies for screening libraries to find a bait interacting polypeptide or peptide. In figure 8A, A is the bait polypeptide or peptide and B varies within the library. In figure 8B, B is the bait polypeptide or peptide and A varies within the library.

### EXAMPLES

In the following description, all molecular biology experiments are performed according to standard protocol (Sambrook et al, 1989).

### 1- Vectors and cell engineering

Yeast 74D694 strong [PSI+] strain (Mata, adel.14, trp-289, his3Δ200, ura3-52, leu2-3,112) was used for all the studies.

### a. Cloning of sup35 gene

Primers sup35a : 5'-CGGGATCCGCATGTCGGATTCAAACCAAG-3' (SEQ ID N°:1) and sup35b : 5'-CGGAATTCGCTTACTCGGCAATTTTAACAATTTTACC-3' (SEQ ID N°:2) containing respectively BamHI and EcoRI restriction sites were used to amplify sup35 gene (2055pb) from total genomic DNA extract from *S. cerevisiae* EYB100 strain. Sup35 was cloned on the pMG71 plasmid between BamHI and EcoRI restriction sites to obtain the pMG-sup35.

### b. Construction of the vector pY-sup35C

The sequence encoding the C terminal domain of sup35 (sup35C) (aa 255-685) was cloned in frame with a linker in a pY plasmid containing the weak PCYC promoter, the URA3 auxotrophic marker and the low copy CEN yeast replication origin. The vector pY-sup35C was constructed from pRS416-PCYC (Mumberg et al, 1995) into which the C terminal domain of sup35 (sup35C) was cloned. For this purpose, PCR amplification with primers fussup35g: 5'-TATACCAAGCTTTTTGGTGGTAAAGATCACG-3' (SEQ ID N°:3) and fus sup35h: 5'-TATACCGCTCGAGCGGTTACTCGGCAATTTTAAC-3' (SEQ ID N°:4) containing respectively HindIII and XhoI restriction sites was performed using pMG-sup35 as template. This PCR fragment was cloned into the pRS416-PCYC plasmid between HindIII and XhoI restriction sites. Fragment containing the linker was PCR amplified from the plasmid p03 using primers fus sup35e: 5'-TATAGGGAATTCAGTGGTGGTGGTTCTGG-3' (SEQ ID N°:5) and fus sup35f: 5'-TATACCCAAGCTTGCGCTTATCGTCATCGTC-3' (SEQ ID N°:6) containing respectively EcoRI and HindIII restriction sites. This fragment was cloned between EcoRI and HindIII restriction sites into pRS416-PCYC-sup35C in frame with sup35C to obtain the pY-sup35C.

### c. Construction of the vector pX-sup35NM

The sequence encoding the N-terminal domain of sup35 (sup35NM) (aa 1-253) was cloned in frame with a linker into the pX plasmid containing a strong PTEF promoter, the TRP-1 auxotrophic marker and the high copy 2µ yeast replication origin. First sup35NM was PCR amplified using pMG-sup35 as template with primers fus sup35a: 5'-TATAGCTCTAGAGCATGTCGGATTCAAACCAAG-3' (SEQ ID N°:7) and fus sup35b: 5'-TATACGACTAGTATCGTTAACAACTTCGTC-3' (SEQ ID N°:8) containing respectively XbaI and SpeI restriction sites. sup35NM was cloned into the PRS416-PTEF yeast plasmid to obtain pTEF-SUP35NM. Fragment containing linker was PCR amplified using primers fus sup35c: 5'-TATACGGACTAGTGGTGGTGGTGGTTC-3' (SEQ ID N°:9) and fus sup35d: 5'-TATAGGGATCCGCGCTTATCGTCATCGTCGTAC-3' (SEQ ID N°:10) containing respectively SpeI and BamHI restriction sites with p03 as template. This linker was then cloned in frame with sup35NM to obtain pTEFsup35NM linker.
To replace the URA3 auxotrophic marker by TRP1 one, SwaI/EcoRI fragment of a plasmid previously construct in the laboratory, the MCS of pMGl-mscpGAL was inserted in place of the SwaI/EcoRI fragment of the PTEFsup35NM linker.

### 2- Development of a reporter system.

In addition of the white/red phenotype a second reporter system was developed to facilitate the detection of colonies containing interacting proteins. A mutated version of the lacZ gene was constructed to allow a blue/white phenotype and positive selection of candidates by flow cytometry.

### a. Example of construction of a mutated reporter gene: the lacZ gene.

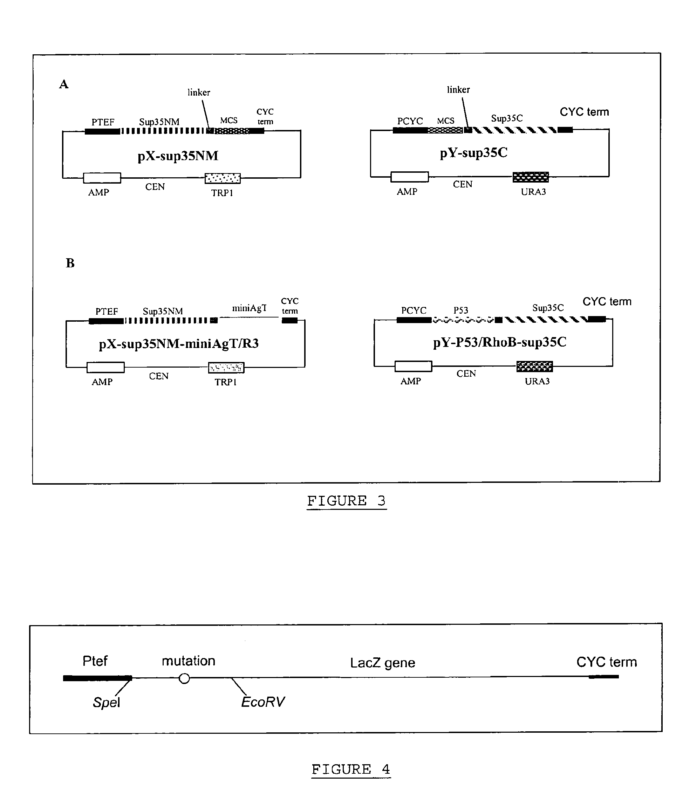

A substitution was made leading to a stop codon in the lacZ ORF. This mutation was performed in order to render lacZ translation dependent on the sup35 prion protein status. With such mutation, when sup35 or sup35C is free, sup35 or sup35C stops the lacZ translation at the internal stop codon. LacZ substrates are not hydrolysed. In contrast, when sup35 or sup35C is entrapped in aggregates, full translation occurs, lacZ mRNA is fully translated and the enzyme produced allows the hydrolysis of X-gal or derivative thereof. The lacZ mutated gene was constructed by overlap PCR. Primers couple: lacZdel1: 5'-GAAAGACTAGTCCAGTGTGGTGGAATTCTGC-3' (SEQ ID N°:11) /lacZdel1' : 5'-TTTCACCCTGTCATAAAGAAACTGTTACCCGTA-3' (SEQ ID N°:12) and lacZdel2: 5'-TACGGGTAACAGTTTCTTTATGACAGGGTGAAAC-3' (SEQ ID N°:13)/lacZdel2' : 5'-CAGCAGGATATCCTGCACCATCGTCTG-3' (SEQ ID N°:14) were used to perform a first PCR step.
lacZdel1' and lacZdel2 overlapping primers contain the mutation which introduce a Stop codon in the codon 257 of lacZ ORF. After this first step, PCR products were used as template for a third PCR step with primers lacZdel1 and lacZdel2' containing SpeI and EcoRV restriction sites to clone the mutated lacZ fragment (cf. fig. 4). The SpeI and EcoRV digested PCR fragment was introduced in order to replace the wild type fragment of the lacZ gene in the pTracer-lacZ plasmid. The entire lacZ gene containing the mutation (lacZmut) was then cloned into the pRS416-TEF yeast expression plasmid.

### b. lacZ-mut translation is function of the sup35 aggregated/soluble status.

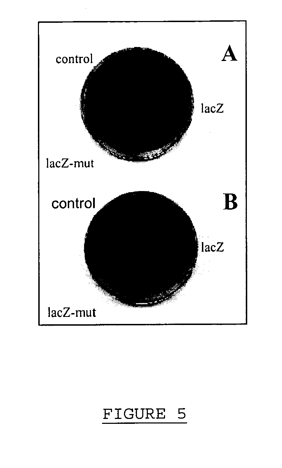

To test the functionality of the mutated lacZ gene in this system, the 74D694 strain was transformed with a control empty plasmid (pRS416-TEF), a plasmid coding the wild type lacZ gene or a plasmid coding the lacZ-mut gene. Transformation products were platted in medium lacking uracile and containing X-gal with or without guanidine hydrochloride (GuHCl). GuHCl was added as control because GuHCl is known to dissociate sup35 aggregates. When GuHcl is added to the medium, sup35 is soluble and stops translation at the premature stop codons.
Results are shown in figure 5. When yeast cells transformed with the wild type lacZ gene are platted in Xgal containing medium or X-gal+GuHCl containing medium, colonies are blue. The gene translation is not stopped at the premature stop codon when sup35 forms part of a protein aggregate. When yeast is transformed with the lacZ-mut gene, colonies are blue when platting in medium containing X-gal and are red in medium containing Xgal+GuHCl. This phenotype change is due to the solubilization of sup35 by GuHCl which can stop translation in premature stop codon (lacZ-mut and ade1.14)
These results show that this lacZ mutant can be used like a reporter system to detect colonies wherein an interaction occurred.

### 3. Study of the intracellular protein interaction of p53 and mini Ag-T

The plasmids pX-sup35NM and pY-sup35C (Fig 3A) were used to clone interacting proteins in order to test the method disclosed in the present invention.
The first couple tested was the well known p53/SV40-AgT which are used as positive interaction control in yeast two hybrid (clontech) and in MagneGST^{™} pull down (promega).

### a. Cloning of p53 gene into the vector pY-sup35C

p53 was cloned in frame with sup35C into the pY-sup35C vector to obtain the pY-P53-sup35C. pY-P53-sup35C was constructed by cloning P53 PCR fragment from pGBKT7/p53 obtained with primers p53_35c_5': 5'-TATATAACTAGTATGCCTGTCACCGAGACCCCT-3' (SEQ ID N°:15) and p53_35c_3': 5'-TATATAGAATTCGTCTGAGTCAGGCCCCACTTTCTT-3' (SEQ ID N°:16) containing SpeI and EcoRI restriction sites respectively in SpeI/EcoRI restriction sites in the pY-sup35C.

### b. Cloning of miniAgTgene into the vector pX-sup35NM

The 880 pb length fragment from SV40AgT containing the P53 binding site was cloned in frame with sup35NM in pX-sup35NM to obtain the pX-sup35NM-miniAgT (Fig. 3B). pX-sup35NM-miniAgT was constructed by cloning mini-AgT PCR fragment from pGADT7/T obtained with primers 35N_miniAgT_5': 5'-TATATAACTAGTATGTTAGCTAAAAAGCGGGTTGATAGCC-3' (SEQ ID N°:17) and 35N_miniAgT_3': 5'-TATATAGAATTCTTAATCTAAAACTCCAATTCCCATAGCCAC-3' (SEQ ID N°:18) containing BamHI and EcoRI restriction site respectively in BamHI/EcoRI in the pX-sup35NM.

### c. Test of the ability of P53-sup35C fusion to reverse [PSI+] phenotype in 74D694 strain.

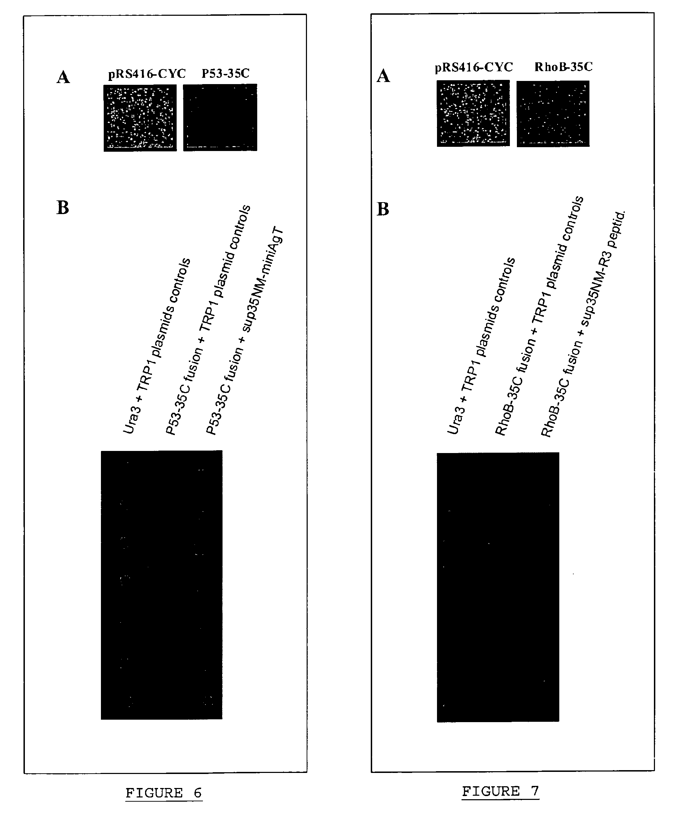

The strain 74D694 was transformed with pY-P53-sup35C and cells were plated on minimal medium YNB supplemented with Complete Supplement Mixture (csm) lacking uracile. A control was performed with the pTEF empty plasmid (without the sup35C coding sequence) and containing the URA3 auxotrophic marker. After four days at 30°C, the phenotype of the colonies was observed (cf. Fig. 6A). Control colonies transformed with the empty plasmid have a [PSI+] white/pink colour-based phenotype. Colonies transformed with plasmid coding the P53-sup35C fusion have a [psi-] red colour-based phenotype (cf. Fig. 6A).
These results indicate that sup35C fused with heterologous protein is able to stop translation at the premature STOP codon of adel-14 allele.

### d. Test of the ability of the fusion sup35NM-miniAgT to restore the [PSI+] phenotype.

The 74D694 strain was cotransformed with the two plasmids: pY-P53-sup35C and pX-sup35NM-miniAgT.
Two controls were performed. The first one was a cotransformation of two empty plasmid harbouring TRP1 and URA3 auxotrophic markers to have a control of [PSI+] white/pink colour-based phenotype. The second control was cotransformation of pY-p53-sup35C with a plasmid harbouring TRP1 auxotrophic marker to dispose of the red phenotype control. The phenotypes of the colonies were observed after 4 days on minimal medium supplemented with complete mixture lacking uracile and tryptophan. P53-sup35C fusion protein generated a [psi-] red colour-based phenotype when cotransformed with either an empty plasmid containing the TRP1 auxotrophic marker or a control plasmid encoding sup35NM fused to R3 peptide, a peptide which interacts with RhoB (cf. example 4), which does not interact with p53.
The colonies resulting from cotransformation with plasmids encoding P53-sup35C and sup35NM-miniAgT fusion proteins showed the [PSI+] white/pink colour-based phenotype (figure 6B). This result indicates that p53 interacts with miniAgT and consequently the ADE1 translation ending at the premature stop codon is prevented. Furthermore, the control colonies transformed with the two TRP1 and URA3 empty plasmids had [PSI+] white/pink colour-based phenotype. These results indicate that the system performs well for highlighting intracytoplasmic protein/protein interaction.

### 4. Study of the intracellular protein interaction of RhoB and R3 peptide.

The plasmids pX-sup35NM and pY-sup35C (Fig 3A) were used to clone interacting proteins in order to test the method disclosed in the present invention. The second couple tested was RhoB/R3 peptides which have been described to interact in our laboratory.

### a. Cloning of RhoB gene into the vector pY-sup35C

RhoB cloning sequence was cloned in frame with sup35C in the pY-sup35C vector to obtain the pY-RHOB-sup35C. This vector was constructed by cloning RhoB PCR fragment obtained with primers RhoB_35c_5': 5'-TATAGGGATCCATGGCTTACCCATACGATG-3' (SEQ ID N°:19) and RhoB_35c_3': 5'-TATAGGGAATTCTAGCACCTTGCAGCAGTTG-3' (SEQ ID N°:20). Primers contain SpeI and EcoRI restriction sites respectively to clone the RhoB containing fragment between SpeI/EcoRI restriction sites of the pY-sup35C (cf. Figure 3B) .

### b. Cloning of R3 gene into the vector pX-sup35NM.

The 248pb BamHI/EcoRI length fragment from pMG-R3 containing R3 peptide coding sequence was cloned in frame with sup35NM in pX-sup35NM to obtain the pX-sup35NM-R3 (cf. Figure 3B)

### c. Test of the ability of RHOB-sup35C fusion to reverse [PSI+] phenotype in 74D694 strain.

The strain 74D694 was transformed with pY-RHOB-sup35C and cells were plated on minimal medium YNB supplemented with csm (Complete Supplement Mixture) lacking uracile. A control was performed with the pTEF empty plasmid (without the sup35C encoding sequence) and containing the URA3 auxotrophic marker. After four days at 30°C the phenotype of the colonies was observed (cf. fig 7A). Control colonies transformed with the empty plasmid have a [PSI+] white/pink phenotype. Colonies transformed with plasmid coding the RHOB-sup35C fusion have a [psi-] red phenotype. These results indicate that sup35C fused with heterologous protein is able to stop translation at the premature STOP codon of ade1-14 allele.

### d. Test of the ability of the fusion sup35NM-R3 to restore the [PSI+] phenotype.

The 74D694 strain was cotransformed with the two plasmids: pY-RHOB-sup35C and pX-sup35NM-R3.
Two controls were performed: The first one is cotransformation of two empty plasmid harbouring TRP1 and URA3 auxotrophic markers to have a control of [PSI+] white/pink color phenotype. The second control is a cotransformation of pY-RhoB-sup35C with a plasmid harbouring TRP1 auxotrophic marker to obtain the red phenotype control. The phenotypes of the colonies were observed after 4 days on minimal medium supplemented with complete amino acid mixture lacking uracile and tryptophan.
The fusion RHOB-sup35C protein confers a [psi-] red phenotype when cotransformed with either an empty plasmid containing the TRP1 auxotrophic marker or with a control plasmid coding a fusion between sup35NM and miniAgT, a protein that doesn't interact with RhoB. The colonies resulting from the cotransformation with plasmids coding the fusion proteins RHOB-sup35C and sup35NM-R3 showed the [PSI+] white/pink phenotype (figure 7B). This phenotype indicates that RhoB interacts with R3 and consequently the ADE1 translation ending at the premature stop codon is prevented. Furthermore, the control colonies transformed with the two TRP1 and URA3 empty plasmids had [PSI+] white/pink colour-based phenotype. These results indicate that the system performs well for highlighting intracytoplasmic protein/protein interaction.

### References:

Throughout this application, various references describe the state of the art to which this invention pertains. The disclosures of these references are hereby incorporated by reference into the present disclosure.
- Broder YC, Katz S, Aronheim A. The ras recruitment system, a novel approach to the study of protein-protein interactions. Curr Biol. 1998 Oct 8;8(20):1121-4.
- Chernoff, Yury O. Amyloidogenic domains, prions and structural inheritance: rudiments of early life or recent acquisition? Current Opinion in Chemical Biology, Volume 8, Issue 6, December 2004, Pages 665-671
- Fields, S; Song, O. A novel genetic system to detect protein-protein interactions. Nature, Volume 340, Issue 6230, July 20, 1989, Pages 245-246
- Johnsson, N; Varshavsky, A. Split ubiquitin as a sensor of protein interactions in vivo. Proceedings Of The National Academy of Sciences of The United States of America, Volume 91, Issue 22, October 25, 1994, Pages 10340-10344.
- Mumberg Dominik, Muller Rolf and Funk Martin . Yeast vectors for the controlled expression of heterologous proteins in different genetic backgrounds ● Gene, Volume 156, Issue 1, 14 April 1995, Pages 119-122.
- Remy Ingrid and Michnick Stephen W. A cDNA library functional screening strategy based on fluorescent protein complementation assays to identify novel components of signaling pathways ● Methods, Volume 32, Issue 4, April 2004, Pages 381-388
- Rossi, F; Charlton, C A; Blau, H M. Monitoring protein-protein interactions in intact eukaryotic cells by beta-galactosidase complementation. Proceedings of The National Academy of Sciences Of The United States of America, Volume 94, Issue 16, August 5, 1997, Pages 8405-8410
- Sambrook, J; Fritsch, E.F; Maniatis, T. Molecular cloning, a laboratory manual. Cold spring harbor laboratory press, 1989
- Ventura S, Zurdo J, Narayanan S, Parreno M, Mangues R, Reif B, Chiti F,Giannoni E, Dobson CM, Aviles FX, Serrano L. Short amino acid stretches can mediate amyloid formation in globular proteins: the Src homology 3 (SH3) case. Proc Natl Acad Sci U S A. 2004 May 11;101(19):7258-63.
- Wehrman, Tom; Kleaveland, Benjamin; Her, Jeng-Horng; Balint, Robert F; Blau, Helen M. Protein-protein interactions monitored in mammalian cells via complementation of beta -lactamase enzyme fragments. Proceedings of The National Academy of Sciences of The United States Of America, Volume 99, Issue 6, March 19, 2002, Pages 3469-3474

## Claims

1. A kit comprising:
- a first expression vector comprising a nucleotide sequence encoding a first chimeric polypeptide of formula X-A comprising an aggregation domain, which enables the aggregation in a host cell of said chimeric polypeptide
wherein X is a polypeptide or a peptide and A is a polypeptide or peptide; and
- a second expression vector comprising a nucleotide sequence encoding a second chimeric polypeptide of formula B-Y, wherein B is a polypeptide or peptide and Y is a polypeptide or a peptide, comprising a phenotype-associated functional domain,
wherein when B-Y and X-A are expressed in a host cell the phenotype of said host cell depends on whether B-Y is entrapped or not in an aggregate, B-Y being entrapped if A and B interact with each other.

2. The kit according to claim 1 wherein X comprises an aggregation domain, which enables the aggregation in a host cell of said chimeric polypeptide.

3. The kit according to claim 1 wherein the first expression vector comprises a nucleotide sequence encoding a polypeptide or peptide A comprising an aggregation domain, which enables the aggregation in a host cell of A and wherein X is not present.

4. The kit according to any one of claims 1 to 3 further comprising a host cell wherein when said first and second expression vectors are introduced into said host cell, said host cell expresses chimeric polypeptides X-A and BY. X-A forms part of an aggregate which is present in said host cell and the phenotype of said host cell depends if B-Y is entrapped or not in said aggregate.

5. The kit according to claim 4 wherein said host cell is a yeast cell or a mammalian cell.

6. The kit according to any one of claims 1 to 5 wherein X comprises an aggregation domain selected from the group consisting of the Sup35 amino-terminal domain, a domain which is rich in glutamine and asparagines, a domain consisting of 50 to 400 glutamine residues and an intracellular insoluble domain.

7. The kit according to any one of claims 1 to 6 wherein X is an amyloidogenic polypeptide selected from the group consisting of a prion protein, Sup35, New1, Ure2, PrP, huntingtin, alpha-syneclein and beta-amyloid peptide, fragments and derivatives thereof having aggregating properties.

8. The kit according to any one of claims 1 to 7 wherein Y is selected from the group consisting of a translation termination factor, a transcription factor and an enzyme.

9. The kit according to any one of claims 1 to 8 wherein A or B is an immunoglobulin, or a member of the immunoglobulin super-family, or any fragment thereof.

10. A kit comprising:
a first expression vector comprising a nucleotide sequence encoding a first chimeric polypeptide of formula X-A as described in any one of claims 1 to 9; and
a second expression vector comprising a cloning site which enables the introduction of a nucleotide sequence encoding a peptide or polypeptide B in such a way that the chimeric polypeptide of formula B-Y as described in any one of claims 1 to 9 is expressed in a host cell when said expression vector is introduced into said host cell.

11. A kit according comprising:
a first expression vector comprising a cloning site which enables the introduction of a nucleotide sequence encoding a peptide or polypeptide A in such a way that the chimeric polypeptide of formula X-A as described in any one of claims 1 to 9 is expressed in a host cell when said expression vector is introduced into said host cell; and
a second expression vector comprising a cloning site which enables the introduction of a nucleotide sequence encoding a peptide or polypeptide B in such a way that the chimeric polypeptide of formula B-Y as described in any one of claims 1 to 9 is expressed in a host cell when said expression vector is introduced into said host cell.

12. A kit according comprising:
a first expression vector comprising a cloning site which enables the introduction of a nucleotide sequence encoding a peptide or polypeptide A in such a way that the chimeric polypeptide of formula X-A as described in any one of claims 1 to 9 is expressed in a host cell when said expression vector is introduced into said host cell; and
a second expression vector comprising a nucleotide sequence encoding a second chimeric polypeptide of formula B-Y as described in any one of claims 1 to 9.

13. A kit according to any one of claims 1 to 9 comprising:
a first expression vector comprising a nucleotide sequence encoding a first chimeric polypeptide of formula X-A as described in any one of claims 1 to 9 or a first expression vector comprising a cloning site which enables the introduction of a nucleotide sequence encoding a peptide or polypeptide A in such a way that the chimeric polypeptide of formula X-A as described in any one of claims 1 to 9 is expressed in a host cell when said expression vector is introduced into said host cell; and
a library of second expression vectors each comprising a nucleotide sequence encoding a chimeric polypeptide of formula B-Y as described in any one of claims 1 to 9,
wherein B is a polypeptide or peptide which varies within the library.

14. A kit according to any one of claims 1 to 9 comprising:
a library of first expression vectors each comprising a nucleotide sequence encoding a first chimeric polypeptide of formula X-A as described in any one of claims 1 to 9, wherein A is a polypeptide or peptide which varies within the library; and
a second expression vector comprising a nucleotide sequence encoding a second chimeric polypeptide of formula B-Y as described in any one of claims 1 to 9 or a second expression vector comprising a cloning site which enables the introduction of a nucleotide sequence encoding a peptide or polypeptide B in such a way that the chimeric polypeptide of formula B-Y as described in any one of claims 1 to 9 is expressed in a host cell when said expression vector is introduced into said host cell.

15. A host cell comprising the first and the second expression vector as described in any one of claims 1 to 9.

16. The host cell according to claim 15 wherein said host cell is a yeast cell or a mammalian cell.

17. A kit comprising:
a first haploid host yeast cell comprising the first expression vector as described in any one of claims 1 to 9; and
a second haploid host yeast cell comprising the second expression vector as described in any one of claims 1 to 9,
wherein said first and said second haploid host yeast cells are of opposite mating type.

18. A kit according to claim 17 comprising:
a first haploid host yeast cell comprising the first expression vector as described in any one of claims 1 to 9; and
a library of second haploid yeast host cells, each comprising the second expression vector encoding a chimeric polypeptide of formula B-Y as described in any one of claims 1 to 9, wherein B is a polypeptide or peptide which varies within the library;
wherein said first and said second haploid host yeast cells are of opposite mating type.

19. A kit according to claim 17 comprising:
a library first haploid host yeast cell, each comprising the first expression vector as described in any one of claims 1 to 9, wherein A is a polypeptide or peptide which varies within the library; and
a second haploid yeast host cells comprising the second expression vector encoding a chimeric polypeptide of formula B-Y as described in any one of claims 1 to 9;
wherein said first and said second haploid host yeast cells are of opposite mating type.

20. A method for selecting a peptide or polypeptide B which binds to a bait peptide or polypeptide A comprising the steps of:
a) generating a library of host cells, each expressing the two chimeric polypeptides X-A and B-Y as described in any one claim 1 to 9 and wherein B is a polypeptide or peptide which varies within the library;
b) detecting a change of phenotype within the host cells of the library;
c) isolating from the host cells with a modified phenotype the expression vector encoding B-Y;
d) determining the nucleotide sequence encoding B from the expression vector isolated in step c).

21. A method for producing a peptide or polypeptide B which binds to a bait polypeptide or peptide A comprising the steps of:
a) selecting the peptide or polypeptide B by performing the method of claim 20; and
b) producing B.

22. A method for selecting a peptide or polypeptide A which binds to a bait peptide or polypeptide B comprising the steps of:
a) generating a library of host cells, each expressing the two chimeric polypeptides X-A and B-Y as described in any one claim 1 to 9 and wherein A is a polypeptide or peptide which varies within the library;
b) detecting a change of phenotype within the host cells of the library;
c) isolating from the host cells with a modified phenotype the expression vector encoding X-A;
d) determining the nucleotide sequence encoding A from the expression vector isolated in step c).

23. A method for producing a peptide or polypeptide A which binds to a bait polypeptide or peptide B comprising the steps of:
a) selecting the peptide or polypeptide A by performing the method of claim 22; and
b) producing A.

24. A method for screening compounds interfering with the interaction of two polypeptides A and B comprising the steps of:
a) adding the compound to be screened to a host cell as described in claim 15 or 16 expressing the two chimeric polypeptides X-A and B-Y;
b) detecting a change of phenotype of said host cell.


SEQUENCE LISTING

<110>  MilleGen

<120>  Intracellular method for selecting a peptide or polypeptide which
        binds to a bait peptide or polypeptide

<130>  BEP060128

<160>  20

<170>  PatentIn version 3.3

<210>  1
<211>  29
<212>  DNA
<213>  artificial

<220>
<223>  primer

<400>  1
cgggatccgc atgtcggatt caaaccaag                                          29

<210>  2
<211>  37
<212>  DNA
<213>  artificial

<220>
<223>  primer

<400>  2
cggaattcgc ttactcggca attttaacaa ttttacc                                 37

<210>  3
<211>  31
<212>  DNA
<213>  artificial

<220>
<223>  primer

<400>  3
tataccaagc tttttggtgg taaagatcac g                                       31

<210>  4
<211>  34
<212>  DNA
<213>  artificial

<220>
<223>  primer

<400>  4
tataccgctc gagcggttac tcggcaattt taac                                    34

<210>  5
<211>  29
<212>  DNA
<213>  artificial

<220>
<223>  primer

<400>  5
tatagggaat tcagtggtgg tggttctgg                                          29


<210>  6
<211>  31
<212>  DNA
<213>  artificial

<220>
<223>  primer

<400>  6
tatacccaag cttgcgctta tcgtcatcgt c                                       31


<210>  7
<211>  33
<212>  DNA
<213>  artificial

<220>
<223>  primer

<400>  7
tatagctcta gagcatgtcg gattcaaacc aag                                     33


<210>  8
<211>  30
<212>  DNA
<213>  artificial

<220>
<223>  primer

<400>  8
tatacgacta gtatcgttaa caacttcgtc                                         30


<210>  9
<211>  27
<212>  DNA
<213>  artificial

<220>
<223>  primer

<400>  9
tatacggact agtggtggtg gtggttc                                            27


<210>  10
<211>  33
<212>  DNA
<213>  artificial

<220>
<223>  primer

<400>  10
tatagggatc cgcgcttatc gtcatcgtcg tac                                     33


<210>  11
<211>  31
<212>  DNA

<213>  artificial

<220>
<223>  primer

<400>  11
gaaagactag tccagtgtgg tggaattctg c                                    31


<210>  12
<211>  33
<212>  DNA
<213>  artificial

<220>
<223>  primer

<400>  12
tttcaccctg tcataaagaa actgttaccc gta                                  33


<210>  13
<211>  34
<212>  DNA
<213>  artificial

<220>
<223>  primer

<400>  13
tacgggtaac agtttcttta tgacagggtg aaac                                 34


<210>  14
<211>  27
<212>  DNA
<213>  artificial

<220>
<223>  primer

<400>  14
cagcaggata tcctgcacca tcgtctg                                         27


<210>  15
<211>  33
<212>  DNA
<213>  artificial

<220>
<223>  primer

<400>  15
tatataacta gtatgcctgt caccgagacc cct                                  33


<210>  16
<211>  36
<212>  DNA
<213>  artificial

<220>
<223>  primer

<400>  16
tatatagaat tcgtctgagt caggccccac tttctt                               36

<210>  17
<211>  40
<212>  DNA
<213>  artificial

<220>
<223>  primer

<400>  17
tatataacta gtatgttagc taaaaagcgg gttgatagcc                              40


<210>  18
<211>  42
<212>  DNA
<213>  artificial

<220>
<223>  primer

<400>  18
tatatagaat tcttaatcta aaactccaat tcccatagcc ac                           42


<210>  19
<211>  30
<212>  DNA
<213>  artificial

<220>
<223>  primer

<400>  19
tatagggatc catggcttac ccatacgatg                                         30


<210>  20
<211>  31
<212>  DNA
<213>  artificial

<220>
<223>  primer

<400>  20
tatagggaat tctagcacct tgcagcagtt g                                       31